# EUROPÄISCHE PATENTSCHRIFT

(11) **EP 3 308 765 B1**
(45) Veröffentlichungstag und Bekanntmachung des Hinweises auf die Patenterteilung: **13.07.2022**
(21) Anmeldenummer: 16194165.3
(22) Anmeldetag: 17.10.2016
(51) Int. Cl.: A61K 6/00, C08F 30/02, C07F 9/09

(54) **RADIKALISCH POLYMERISIERBARE VERBINDUNG**
RADICAL POLYMERIZABLE COMPOUND
COMPOSÉ POLYMÉRISABLE PAR VOIE RADICALAIRE

(43) Veröffentlichungstag der Anmeldung: 18.04.2018
(73) Patentinhaber: Mühlbauer Technology GmbH, 22547 Hamburg (DE)
(72) Erfinder: Neffgen, Stephan, 25421 Pinneberg (DE); Becker, Olav-Sven, 20253 Hamburg (DE)
(74) Vertreter: Glawe, Delfs, Moll

(56) Entgegenhaltungen:
- EP-A1- 1 674 066
- EP-A1- 2 527 321
- EP-A1- 2 662 067
- WO-A1-2013/083734
- DE-B3- 10 239 204
- JP-A- 2007 161 622
- KR-A- 20150 028 464
- US-A1- 2015 132 544
- US-B2- 8 404 144
- Araceli G. Campaña ET AL: "One-Dimensional Random Walk of a Synthetic Small Molecule Toward a Thermodynamic Sink", JOURNAL OF THE AMERICAN CHEMICAL SOCIETY, vol. 135, no. 23, 30 May 2013 (2013-05-30) , pages 8639-8645, XP055709646, US ISSN: 0002-7863, DOI: 10.1021/ja402382n
- Matthew R Lashley ET AL: "Synthesis and estrogen receptor affinity of a 4-hydroxytamoxifen-Labeled ligand for diagnostic imaging", Bioorganic & medicinal chemistry : a Tetrahedron publication for the rapid dissemination of full original research papers and critical reviews on biomolecular chemistry, medicinal chemistry and related disciplines, vol. 10, no. 12, 1 December 2002 (2002-12-01), pages 4075-4082, XP055709662, NL ISSN: 0968-0896, DOI: 10.1016/S0968-0896(02)00329-2

## Beschreibung

Die Erfindung betrifft radikalisch polymerisierbare Verbindungen, Verfahren zu deren Herstellung, die Verwendung der Verbindungen in einem Dentalmaterial, insbesondere als Bestandteil eines Haftvermittlers, sowie polymerisierbare Dentalmaterialien, die diese Verbindungen enthalten.

Es sind zahlreiche radikalisch polymerisierbare Verbindungen zur Verwendung als Haftvermittler enthaltend auf der einen Seite eine Säuregruppe, bspw. eine 1,1-Bisphosphonsäuregruppe und auf der anderen Seite eine radikalisch polymerisierbare Gruppe, bspw. eine (Meth)acrylatgruppe bekannt.

In der EP1296634B1 (Erdmann et al.) und US8404144B2 (Abuelyaman et al.) werden hydrolysestabile 1,1-Bisphosphonsäuren zur Verwendung in Dentalmaterialien beschrieben, die als Abstandsgruppen lineare Alkylengruppen und eine sich daran anschließende (Meth)acrylamidgruppe aufweisen.

In der EP2662067 (Klee et al.) werden lineare Polyethyleniminderivate beschrieben die polymerisierbare Gruppen und/oder Carboxylgruppen ausschließlich in den Seitenketten aufweisen. Die Polyethyleniminderivate sind besonders zur Verwendung in harzverstärkten Glasionomerzementen geeignet.

In der EP2489344A1 (Salz et al.) werden nicht polymerisierbare antimikrobielle Wirkstoffe für polymerisierbare Dentalmaterialien beschrieben, die eine Ankergruppe und einen Spacer, der eine Polyethylenimingruppe oder eine N-alkylierte Polyethylenimingruppe sein kann, aufweisen können.

DE 102 39 204, Campaña et. al., One-Dimensional Random Walk of a Synthetic Sma11 Molecule Toward a Thermodynamic Sink, J. Am. Chem. Soc., 2013, 135, 8639-8645, Lashley et. al., Synthesis and estrogen receptor affinity of a 4-hydroxytamoxifen-Labeled ligand for diagnostic imaging, BioMed. Chem., 2002, 10, 4075-4082 und EP 2 527 321 A1 offenbaren Verbindungen, in denen als Säuregruppe Carbonsäure-, tert-Butylcarbonyl-, Sulfonsäure- oder Sulfonatgruppen vorhanden sind.

US 8 404 144 und WO 2013/083734 betreffen Zusammensetzungen, die polymerisierbare Bisohosphonsäuren enthalten.Verbindungen, in denen eine Abstandsgruppe A Polyethyleniminderivatgruppen aufweisen, werden nicht offenbart.

Der Erfindung liegt die Aufgabe zugrunde, Verbindungen der eingangs genannten Art zu schaffen, die in oder als Bestandteil von polymerisierbaren Dentalmaterialien vorteilhafte Eigenschaften aufweisen, vorzugsweise eine gute Haftung an der Zahnsubstanz (Dentin und/oder Schmelz) vermitteln.

Gelöst wird diese Aufgabe durch eine radikalisch polymerisierbare Verbindung der Formel I:

(SG)ₓA((PEI)TG)ₘ

mit
SG = -OPO₃R¹₂ oder _PO₃R¹₂;
R¹ = unabhängig voneinander H, C₁-C₇-Alkyl oder monovalentes Kation, bevorzugt H;
x = 2;
A: Kohlenwasserstoffgruppe mit 1 bis 30 Kohlenstoffatomen, die Silicium, Halogen, Stickstoff, Phosphor, Sauerstoff und Schwefel enthalten kann; 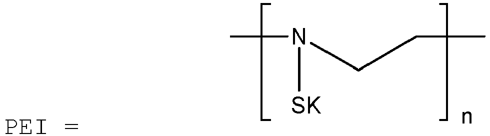
SK = H, C₁-C₂₀-Alkyl, Aryl, Alkyl-Aryl, - (CO) NR³R⁴, - (CS)NR³R⁴, -(CO)OR³, -(CO)R³ oder -(SO₂)R³;
R³, R⁴ = unabhängig voneinander H, Alkyl, Aryl und/oder Alkyl-Aryl, welche Halogen, Stickstoff, Phosphor, Sauerstoff und Schwefel enthalten können oder Alkenyl;
n = 2 - 100, bevorzugt 3 bis 30, besonders bevorzugt 5 bis 15;
TG = H, -NR⁶R⁷, -N₃, -OR⁶, -SR⁶, Cycloalkene, -CR⁶R⁷R⁸, -OCOR⁹ oder -NR⁶COR⁹, bevorzugt -NR⁶R⁷oder -OR⁶, besonders bevorzugt -OCOR⁹ oder -NR⁶COR⁹;
R⁶, R⁷, R⁸ = H, C₁ bis C₂₀-Alkyl, Aryl und/oder Alkyl-Aryl, welche Halogen, Stickstoff, Phosphor, Sauerstoff und Schwefel enthalten können; eine radikalisch polymerisierbare Gruppe, die Halogen, Stickstoff, Phosphor, Sauerstoff und Schwefel enthalten kann, bevorzugt mit 1 bis 20 Kohlenstoffatomen; wobei R⁶ und R⁷ unter Einschluss des Stickstoffatoms einen Ring mit 5 bis 7 Ringatomen bilden können, der Heteroatome enthalten kann;
R⁹ = Alkyl, Aryl, Alkyl-Aryl oder Alkenyl, bevorzugt mit 1 bis 19 Kohlenstoffatomen, besonders bevorzugt mit 1 bis 10 Kohlenstoffatomen;
wobei PEI mehrere radikalisch polymerisierbare Gruppen enthält und TG optional mindestens eine radikalisch polymerisierbare Gruppe enthält;
m = 1, 2 oder 3, bevorzugt 1 oder 2.

Die Bestandteile der genannten Formel I werden nachfolgend kurz erläutert.

SG ist eine Säuregruppe oder deren Ester oder Salz, bevorzugt eine Säuregruppe, die die Fähigkeit besitzt, an einer mineralischen Oberfläche zu haften. Bevorzugte Säuregruppen sind Phosphonsäuregruppen.. SG ist bevorzugt ausschließlich an die unten näher erläuterte Abstandsgruppe A gebunden. Die Anzahl der an die Abstandsgruppe A gebundenen Säuregruppen ist x gleich 2. Bevorzugt sind die Säuregruppen an direkt benachbarte C-Atome gebunden, besonders bevorzugt am selben C-Atom. Bevorzugt ist eine Säuregruppe an ein C-Atom gebunden, das innerhalb des Moleküls am weitesten von der Polyethyleniminderivatgruppe oder der radikalisch polymerisierbaren Gruppen entfernt liegt.

A ist eine Abstandsgruppe zwischen der Säuregruppe SG und den polymerisierbaren Gruppen bzw. zwischen der Säuregruppe und der Polyethyleniminderivatgruppe PEI. Die polymerisierbaren Gruppen sind Bestandteil wenigstens einer der beiden unten näher erläuterten Gruppen PEI (Polyethyleniminderivatgruppe) oder TG (terminale bzw. organische Gruppe).

Es ist bevorzugt, dass A eine Alkylen- oder Aryl-Alkylen- bzw. Alkyl-Arylengruppe ist, bevorzugt mit 1 bis 20 Kohlenstoffatomen, weiter bevorzugt mit 6 bis 20 Kohlenstoffatomen, besonders bevorzugt eine lineare C₂-C₁₄-Alkylengruppe.

PEI ist eine Polyethyleniminderivatgruppe. Die Anzahl der an die Abstandsgruppe A gebundenen Polyethylenimminderivatgruppen ist m gleich 1 bis 3, bevorzugt 1 oder 2.

Die Polyethyleniminderivatgruppe weist einen zahlenmittleren Polymerisationsgrad n von 2 bis 100, bevorzugt 3 bis 30, besonders bevorzugt 5 bis 15 auf.

Die Polyethyleniminderivatgruppe weist mindestens eine Seitenkette SK auf, die nicht H ist, bevorzugt mehrere solcher Seitenketten SK, weiter bevorzugt eine Anzahl solcher Seitenketten SK, die dem mittleren Polymerisationsgrad n entspricht.

SK ist eine Seitenkette der Polyethyleniminderivatgruppe. Bevorzugt weist die mindestens eine Seitenkette mindestens eine radikalisch polymerisierbare Gruppe auf, weiter bevorzugt enthalten mehrere Seitenketten mindestens eine radikalisch polymerisierbare Gruppe.

Sind die Seitenketten SK unterschiedliche Gruppen p, r etc., so sind diese statistisch am PEI verteilt.

Bevorzugte Seitenketten SK enthalten eine direkt an ein Stickstoffatom gebundene (CO)N-, (CS)N-, (CO)O-, (CO)- oder (SO2)-Gruppe, bzw. sind aus dieser ausgewählt. Besonders bevorzugt ist die (CO)-Gruppe.

Bevorzugt mindestens 50 %, weiter bevorzugt mindestens 80 %, weiter bevorzugt mindestens 90 % aller Seitenketten SK und besonders bevorzugt alle Seitenketten SK weisen eine solche Gruppe auf.

Bevorzugt enthält die Seitenkette SK keine Säuregruppe SG.

Ist die Seitenkette SK und/oder R⁶, R⁷ gleich H, Alkyl, Aryl oder Alkyl-Aryl, kann die Polyethylenimineinheit bzw. TG zusätzlich protoniert vorliegen. Bevorzugt weist die korrespondierende Säure zum Gegenion protonierter Gruppen eine größere Säurestärke auf als die Säuregruppe SG, bevorzugt ist die korrespondierende Säure HCl.

Besonders bevorzugte Seitenketten SK sind (Meth)acryloyl- und C5 bis C10-Alkanoyl-Gruppen.

TG ist eine organische Endgruppe oder terminale Gruppe. Die Endgruppe TG kann je nach Anforderung unterschiedliche funktionelle Gruppen aufweisen. Die Endgruppe TG kann gesättigte oder ungesättigte, offenkettige oder cyclische Kohlenwasserstoffgruppen, Amine, Ether, OH-, Amid- oder Estergruppen enthalten.

R³, R⁴ sind bevorzugt ausgewählt aus der Gruppe bestehend aus C₁ bis C₁₉-Alkyl-, bevorzugt C₅ bis C₁₀-Alkyl und C₂-C₁₉-Alkenyl, bevorzugt C₂ bis C₉-Alkenyl, weiter bevorzugt - CR⁵CH₂; mit R⁵ = H oder C₁-C₇-Alkyl, bevorzugt H oder -CH₃, besonders bevorzugt H.

In einer besonders bevorzugten Ausführungsform enthält TG eine Amid- oder Estergruppe.

Die Endgruppe TG ist bevorzugt eine radikalisch polymerisierbare Gruppe bzw. weist eine solche auf.

Optional kann mindestens eine Endgruppe TG mindestens eine polymerisierbare Gruppe aufweisen. Bevorzugte polymerisierbare Gruppen sind Alkenylgruppen. Besonders bevorzugt sind (Meth)acrylamid- oder (Meth)acrylat-Gruppen. Die erfindungsgemäße Verbindung weist somit in dem Bestandteil PEI mehrere radikalisch polymerisierbare Gruppen auf und kann in dem Bestandteil TG wenigstens eine polymerisierbare Gruppe aufweisen.

Die Erfindung stellt radikalisch polymerisierbare Verbindungen zur Verfügung, deren physikalische und chemische Eigenschaften durch die Ausgestaltung der wesentlichen Bestandteile SG, A, PEI und TG leicht an den gewünschten Verwendungszweck angepasst werden können. Die Verbindungen können in Dentalmaterialien eine hohe Haftkraft aufweisen bzw. vermitteln und diese dauerhaft beibehalten. Die Verbindungen sind hydrolysestabil und damit langzeitbeständig sowohl bei der Lagerung als auch nach der Anwendung im oralen Milieu. Mittels der erfindungsgemäßen Verbindungen lassen sich daher einteilige Dentalmaterialien (Einkomponentensysteme) zur Verfügung stellen, bei denen keine Mischung mehrerer Komponenten durch den Anwender erforderlich ist. Die Dentalmaterialien können selbstätzend und/oder selbstprimend ausgebildet sein, eine Vorbehandlung durch Ätzmittel, Primer oder Haftvermittler ist nicht erforderlich. Die erfindungsgemäßen Verbindungen sind bevorzugt löslich in dem Dentalmaterial, in dem sie zur Anwendung kommen.

Eine bevorzugte Ausführungsform der Erfindung besitzt die Formel II: 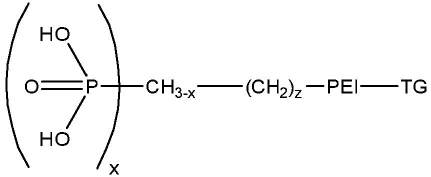 mit
x = 2;
z = 2 bis 14.

Eine weitere bevorzugte Ausführungsform der Erfindung besitzt die Formel III: 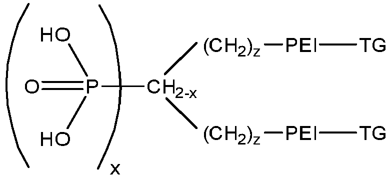 mit
x = 2;
z = 2 bis 14, bevorzugt 2 bis 9 oder weiter bevorzugt 2 bis 6.

Nachfolgend werden beispielhaft einige erfindungsgemäß bevorzugte Verbindungen gezeigt:

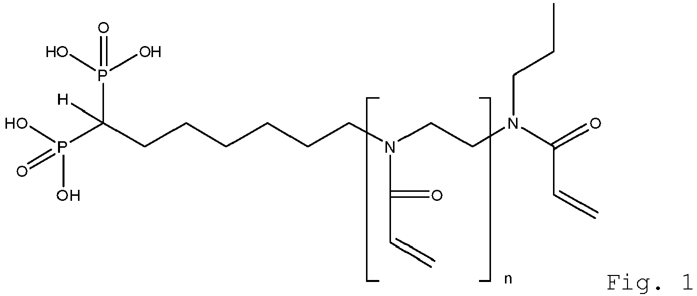

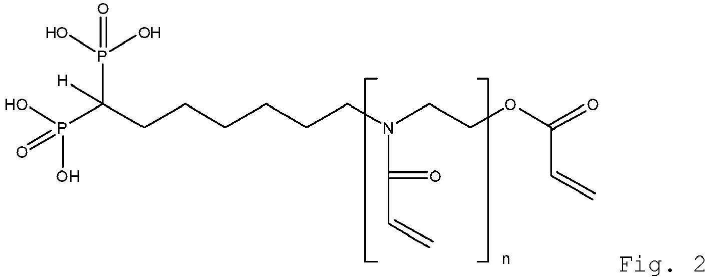

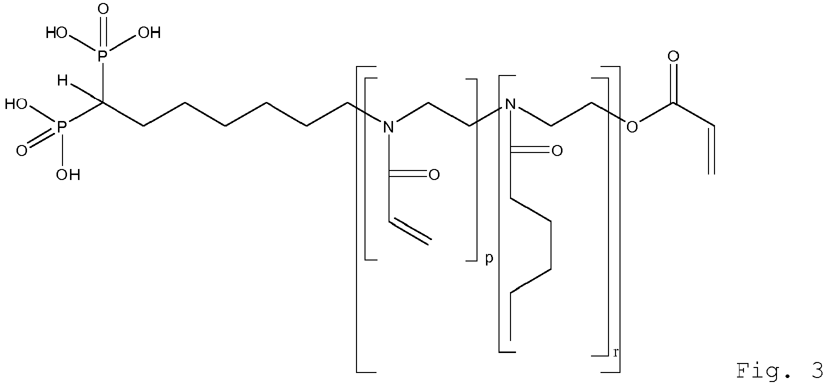

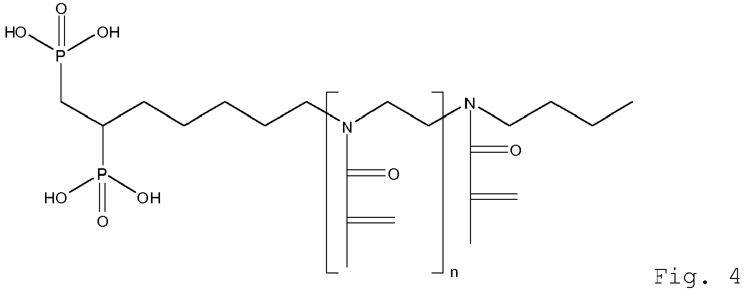

Ein weiterer Gegenstand der Erfindung ist ein Verfahren zur Herstellung erfindungsgemäßer Verbindungen. Die Synthese der radikalisch polymerisierbaren Verbindung nach Formel I erfolgt gemäß dem erfindungsgemäßen Verfahren über eine kationische Polymerisation von Oxazolinen.

Bevorzugt erfolgen anschließend die Hydrolyse des entstandenen Polyoxazolins und eine Derivatisierung entstandener Ethylenimineinheiten.

In einer ersten bevorzugten Ausführungsform wird über den Polymerisationsinitiator dieser kationischen Polymerisation die Säuregruppe SG eingebracht. Hierfür weist der Polymerisationsinitiator mindestens eine Säurederivatgruppe und mindestens eine die kationische Oxazolinpolymerisation initiierende Gruppe auf. Die Säurederivatgruppe wird so ausgewählt, dass sie die Oxazolinpolymerisation nicht startet oder stört, insbesondere ist die Säurederivatgruppe SG hier bevorzugt keine Sulfonsäuregruppe oder Sulfonsäurederivatgruppe.

Bei den initiierenden Gruppen handelt es sich um elektrophile Gruppen, wie organische Halogenide, insbesondere Bromide und Iodide oder Sulfonsäureester etc. Die die kationische Oxazolinpolymerisation initiierende Gruppe ist über die Abstandsgruppe A mit der Säuregruppe SG verbunden. Die initiierende Gruppe wird während der Initiierung und Polymerisation durch eine Polyoxazolingruppe ersetzt.

Bevorzugte Oxazoline sind Alkyl- und Aryloxazoline.

Die kationische Polymerisation der Oxazoline wird mit einer Abbruchverbindung enthaltend eine geeignete nukleophile Gruppe beendet. Geeignete nucleophile Gruppen sind dem Fachmann bekannt, bspw. primäre oder sekundäre Amine, Hydroxidionen oder Carbanionen. Mit der Abbruchverbindung können die Endgruppen TG bzw. Vorstufen dieser Gruppen in die radikalisch polymerisierbaren Verbindungen nach Formel I eingeführt werden. Mit der Wahl der Abbruchverbindung kann die Endgruppe TG gezielt variiert werden.

Bevorzugte nucleophile Gruppen der Abbruchverbindungen sind Amingruppen und Hydroxylgruppen.

Das entstandene Polyalkyloxazolin kann vollständig oder teilweise zum Polyethylenimin hydrolysiert werden.

Die sekundären Aminogruppen des Polyethylenimins können mit allen Verbindungen derivatisiert werden, die mit sekundären Aminogruppen reagieren können. Bevorzugt sind solche, die eine Amid-Gruppierung bilden, bspw. Säurechloride oder Säureanhydride. Zur Einführung von radikalisch polymerisierbaren Gruppen sind besonders (Meth)acrylsäurechloride geeignet.

In einer zweiten bevorzugten Ausführungsform wird über den Polymerisationsinitiator der kationischen Polymerisation die Endgruppe TG (in analoger Weise zur Säuregruppe SG) und die Säuregruppe SG (in analoger Weise zur Endgruppe TG) über die Abbruchverbindung eingebracht. Die Abbruchverbindung enthält dann eine Säuregruppen SG und die Abstandsgruppe A.

Als nucleophile Gruppen sind Aminogruppen, Hydroxylgruppen oder auch Carbanionen geeignet, bevorzugt Anionen CH-acider Substanzen, wie Enolate oder heteroanaloge Enolate.

Besonders bevorzugte Abbruchverbindungen sind **α**-deprotonierte Methylenbisphosphonsäuretetraalkylester oder Derivate von Aminoalkylbisphosphonsäuren, wie z.B. die Derivate der Alendronsäure.

Beide bevorzugten Ausführungsformen sind einfach umzusetzen und die Eigenschaften der resultierenden Verbindungen können gezielt eingestellt werden. Zum Beispiel kann die Hydrophilie der Verbindungen über den Polymerisationsgrad und/oder die Art der Derivatisierung der Polyethylenimingruppe und/oder die Zahl der Seitenketten in der Polyethyleniminderivatgruppe gesteuert werden. Diese Steuerung kann unabhängig von der Wahl der Säuregruppe SG und/oder der Wahl der Endgruppe TG erfolgen. Für die Einführung der Säuregruppe SG und der Endgruppe TG in die resultierende Verbindung steht zudem eine große Auswahl an Verbindungen zur Verfügung.

Ein weiterer Gegenstand der Erfindung ist die Verwendung einer erfindungsgemäßen Verbindung als Bestandteil von bzw. in einem Dentalmaterial, insbesondere einem radikalisch polymerisierbaren Dentalmaterial.

Erfindungsgemäß sollen unter radikalisch polymerisierbaren Dentalmaterialen Materialien zur biomedizinischen Verwendung an Zahnhartsubstanz, insbesondere an Zahnschmelz und Dentin und am Knochengewebe, insbesondere am Kieferknochen verstanden werden.

Die erfindungsgemäßen radikalisch polymerisierbaren Verbindungen nach Formel I sind besonders geeignet als Haftvermittler zwischen mineralischen Oberflächen und radikalisch polymerisierbaren Materialien.

Erfindungsgemäß bevorzugte Dentalmaterialien sind Materialien, die selbst als Restaurationsmaterial, zur Vorbereitung einer Restauration oder zur Verbindung eines Restaurationsmaterials und/oder Zahnrestaurationen aus anorganischen Materialien wie Oxidkeramiken, Silikatkeramiken oder Metallen, bevorzugt Oxidkeramiken mit Zahnhartsubstanz verwendet werden.

Ein weiterer Gegenstand der Erfindung ist ein Dentalmaterial, das enthält:
a) mindestens eine erfindungsgemäße Verbindung;
b) mindestens ein mit a) radikalisch copolymerisierbares Monomer;
c) wenigstens einen Initiator für die radikalische Polymerisation;
d) optional Lösungsmittel;
e) optional Füllstoffe;
f) dentalübliche Additive.

Das erfindungsgemäße Material kann als Einkomponentensystem ausgebildet sein, d.h. es müssen vor der Anwendung keine zwei oder mehr Komponenten des Dentalmaterials getrennt voneinander gelagert und miteinander gemischt werden.

Das erfindungsgemäße Dentalmaterial ist bevorzugt licht-, chemisch oder dual härtend.

Das erfindungsgemäße Dentalmaterial kann bevorzugt in einem Schritt angewendet werden, d.h. es müssen vor der Anwendung keine Vorbereitungsschritte wie Ätzen oder Primen durchgeführt werden.

Das erfindungsgemäße Dentalmaterial ist somit bevorzugt selbstätzend, d.h. es muss vor der Anwendung keine Konditionierung der Zahnhartsubstanz umfassend einen Ätzschritt erfolgen. Dies bedeutet, dass die Klebefläche der Zahnhartsubstanz (insbesondere des Dentins) nicht in einem separaten Schritt mittels Säure geätzt zu werden braucht, um gute Haftwerte zu erzielen.

Das erfindungsgemäße Dentalmaterial ist bevorzugt selbstprimend, d.h. es muss vor der Anwendung kein Primer oder zusätzlicher Haftvermittler angewendet werden.

In einer weiteren Variante der Erfindung kann das erfindungsgemäße Dentalmaterial als Mehrkomponenten-System ausgebildet sein. Gegenstand der Erfindung ist dann auch ein zwei- oder mehrteilige Kit aus diesen Komponenten zur Herstellung eines Dentalmaterials durch Mischen dieser Komponenten.

Die Ausbildung als Mehrkomponenten-System kann insbesondere bevorzugt sein für chemisch- oder dualhärtende Systeme, d.h. wenn eine Lichthärtung schwierig oder nicht möglich ist. Sie kann ferner bevorzugt sein zur nochmaligen Verbesserung der Lagerstabilität, da miteinander weniger oder nicht kompatible Bestandteile getrennt werden können.

Es kann bevorzugt sein, dass sämtliche radikalisch polymerisierbare Verbindungen gemäß Formel I eines mehrteiligen (mehrkomponentigen) Dentalmaterials in einer ersten Komponente des Dentalmaterials enthalten sind.

In einer zweiten Komponente des Dentalmaterials sind bevorzugt Wasser oder wasserreiche Komponenten und/oder von erfindungsgemäßen Verbindungen verschiedene radikalisch polymerisierbare Monomere und/oder Initiator für die radikalische Polymerisation enthalten.

Ebenso sind bevorzugt säureempfindliche Verbindungen bzw. Verbindungen, die mit der Säuregruppe der radikalisch polymerisierbare Verbindungen gemäß Formel I unter Bildung nachteiliger Produkte reagieren (bspw. Salzbildung mit Aminen) in einer zweiten Komponente des Dentalmaterials enthalten.

Nachteilig für die Lagerbarkeit der erfindungsgemäßen Materialien kann es sein, wenn Metallverbindungen, insbesondere Schwermetallverbindungen, in Komponenten enthalten sind, die gleichzeitig Reduktions- oder Oxidationsmitteln enthalten. Dabei möglicherweise auftretende unerwünschte Reaktionen sind dem Fachmann prinzipiell bekannt. Bespielhaft genannt sei die katalytische Zersetzung von Peroxiden durch Schwermetallverbindungen, die auch zur Selbsterhärtung der jeweiligen Komponenten führen kann, oder die Reduktion von Schwermetallverbindungen zu elementaren Metallen oder unwirksameren Verbindungen niedrigerer Oxidationsstufen durch starke Reduktionsmittel wie Sulfinatsalze. Überdies sei die bekannte Autooxidationsreaktion von CH-aciden Stoffen in Gegenwart von Cu2+ genannt.

In mehrkomponentigen Materialien sind daher Schwermetallverbindungen bevorzugt in Komponenten enthalten, die keine Peroxide, keine CH-aciden Verbindungen oder auch keine starken Reduktionsmittel enthalten, die mit den Schwermetallverbindungen reagieren können.

Ebenfalls nachteilig für die Lagerbarkeit der erfindungsgemäßen Materialien können Reaktionen starker Nucleophile, bspw. aus der Stoffgruppe c) (Initiator für die radikalische Polymerisation) stammend, mit den elektronenarmen Doppelbindungen der Bestandteile aus den Stoffgruppen a) und b) sein. Exemplarisch genannt sei die Reaktion von Sulfinaten mit (Meth)acrylaten oder anderen elektrophilen ungesättigten Gruppen, die zur chemischen Veränderung der Formulierung führen.

Es ist daher bevorzugt, dass starke Nucleophile nicht zusammen mit den Bestandteilen aus den Stoffgruppen a) und b) in einer Komponente enthalten sind.

In einer weiteren bevorzugten Ausführungsform, in der ein starkes Nukleophil in einer Komponente zusammen mit Bestandteilen aus den Stoffgruppen a) oder b) enthalten sein kann, ist das starke Nukleophil nicht löslich in der flüssigen Mischung, in denen Bestandteile aus den Stoffgruppen a) oder b) enthalten sind.

Bevorzugte Bestandteile b) bis f) eines erfindungsgemäßen Dentalmaterials werden nachfolgend offenbart.

### Bestandteil b) - mit der radikalisch polymerisierbaren Verbindung nach Formel I radikalisch copolymerisierbares Monomer

Das erfindungsgemäße Dentalmaterial enthält bevorzugt mit der radikalisch polymerisierbaren Verbindung nach Formel I radikalisch copolymerisierbare Monomere.

Die radikalisch copolymerisierbaren Monomere weisen bevorzugt mindestens eine Acrylat- und/oder mindestens eine Methacrylatgruppe, weiter bevorzugt mindestens zwei Acrylat- und/oder mindestens zwei Methacrylatgruppen auf.

Weiter bevorzugt enthält das erfindungsgemäße Dentalmaterial radikalisch copolymerisierbare Monomere, die nur eine Acrylat- oder Methacrylatgruppe aufweisen und radikalisch polymerisierbare Monomere, die mindestens zwei Acrylat- oder Methacrylatgruppen aufweisen.

Geeignete Monomere mit einer Acrylat- oder Methacrylatgruppe sind beispielsweise Hydroxyethyl(Meth)Acrylat, Hydroxypropyl(Meth)Acrylat, Hydroxybutyl(Meth)Acrylat, Glycerin-mono(Meth)Acrylat, Methyl(Meth)Acrylat, Ethyl(Meth)Acrylat, Propyl(Meth)Acrylat, Butyl(Meth)Acrylat, Hexyl(Meth)Acrylat, Octyl(Meth)Acrylat, Lauryl(Meth)Acrylat, Decyl(Meth)Acrylat, Tridecyl(Meth)Acrylat, 2-Ethoxyethyl(Meth)Acrylat, 2'-Ethoxy-2-Ethoxyethyl(Meth)Acrylat, Polyethylenglycolmono(Meth)Acrylat, Polypropylenglycolmono(Meth)Acrylat, Polytetramethyl-englycolmono(Meth)Acrylat. Bevorzugt sind Hydroxyethyl(Meth)Acrylat, Hydroxypropyl(Meth)Acrylat, Hydroxybutyl(Meth)Acrylat, Glycerinmono(Meth)Acrylat und Polyethyl-englycolmono(Meth)Acrylat.

Geeignete Monomere mit mindestens zwei Acrylat- oder Methacrylatgruppen sind beispielsweise 1,3-Propandioldi(meth)acrylat; 1,3-Butandioldi(meth)acrylat; 1,4-Butandioldi(meth)acrylat; 1,5-Pentandioldi(meth)acrylat; Neopentylglykoldi(meth)acrylat; 1,6-Hexandioldimethacrylat; 1,9-Nonandi-oldi(meth)acrylat; 1,10-Decandioldi(meth)acrylat; 1,12-Dode-candioldi(meth)acrylat; Glycerindi(Meth)Acrylat, Ethylenglykoldi(meth)acrylat; Diethylenglykoldi(meth)acrylat; Triethyl-englykoldi(meth)acrylat; Propoxyliertes (2) Neopentylglykoldi(meth)acrylat; Bisphenol-A-di(meth)acrylat; Bisphenol-A-glyceroldi(meth)acrylat (BisGMA); Ethoxyliertes Bisphenol-A-Di(meth)acrylat; Propoxyliertes Bisphenol-A-Di(meth)acrylat; Diurethandi(meth)acrylat (UDMA); Tricyclo[5.2.1.0]decan-dimethanoldi(meth)acrylate; Bis[2-(2-methylacryl-amino)-ethoxycarbonyl]-hexamethylendiamin; Trimethylolpropan-tri(meth)acrylat; Di-Trimethylolpropan-tetra(meth)acrylat; Di-Pentaerythritol-penta(meth)-acrylat; Di-Pentaerythritolhexa(meth)acrylat; sowie die polyalicyclischen Strukturen, die in EP2436363; EP2436366; EP2436388; EP2450025; US8697772 ; US8669302; WO2013023138 beschrieben werden.

Bevorzugt sind Bisphenol-A-di(meth)acrylat; Bisphenol-A-glyceroldi(meth)acrylat (BisGMA); Ethoxyliertes Bisphenol-A-Di(meth)acrylat; Propoxyliertes Bisphenol-A-Di(meth)acrylat; Diurethandi(meth)acrylat (UDMA) und Tricyclo[5.2.1.0]decan-di-methanoldi(meth)acrylate.

Bevorzugt ist das Verhältnis radikalisch copolymerisierbarer Monomere, die nur eine Acrylat- oder Methacrylatgruppe aufweisen zu radikalisch copolymerisierbaren Monomeren, die mindestens zwei Acrylat- oder Methacrylatgruppen aufweisen im Dentalmaterial 0 zu 1 bis 1 zu 1.

Bevorzugt enthält das Material für den Fall, dass das Verhältnis (Mono(Meth)Acrylat) : höherfunktionelles (Meth)Acrylat > 0 ist, aus der Gruppe der Monomere mit einer (Meth)Acrylatgruppe mindestens ein (Meth)Acrylat, das eine substantielle Wasserlöslichkeit besitzt und als Phasenvermittler zwischen stark wasserhaltigen Bereichen der Substrate mit schlecht wasserlöslichen Anteilen der Stoffgruppen a) und b) fungieren kann.

In einer weiteren Ausführungsform enthält das Material eine substantiell wasserlösliche Substanz aus der Gruppe der höherfunktionellen Methacrylate. Substantiell wasserlösliche Substanzen haben bevorzugt eine Löslichkeit in Wasser bei 23°C von mehr als 20 g/L.

Das erfindungsgemäße Dentalmaterial enthält bevorzugt ein mit der radikalisch polymerisierbaren Verbindung nach Formel I radikalisch copolymerisierbares Monomer enthaltend Polyoxyalkylengruppen mit mehr als vier Oxyalkyleneinheiten.

Das erfindungsgemäße Dentalmaterial kann weitere radikalisch polymerisierbare Monomere, Oligomere und Präpolymere enthalten.

Das erfindungsgemäße Dentalmaterial enthält bevorzugt kein mit der radikalisch polymerisierbaren Verbindung nach Formel I radikalisch copolymerisierbares Monomer enthaltend eine andere radikalisch polymerisierbare Gruppe, als eine Acrylat- und/oder Methacrylatgruppe, insbesondere keine Acrylamid- und/oder Methacrylamidgruppe.

Mischungen aus Komponenten, die Acrylat- und/oder Methacrylatgruppen enthalten, zeichnen sich zum einen durch eine gute Reaktivität für radikalische Polymerisationen und zum anderen durch eine breite kommerzielle Verfügbarkeit aus.

Das erfindungsgemäße Dentalmaterial enthält bevorzugt kein mit der radikalisch polymerisierbaren Verbindung nach Formel I radikalisch copolymerisierbares Monomer enthaltend eine Säuregruppe.

### Bestandteil c) - Initiator für die radikalische Polymerisation

In einer Ausführungsform enthält das erfindungsgemäße Dentalmaterial Photoinitiatoren bzw. Photoinitiator-Systeme, bevorzugt für einen Wellenlängenbereich des Lichtes von 390 nm bis 500 nm geeignete Photoinitiatoren bzw. Photoinitiator-Systeme. Diese sind dem Fachmann bekannt.

Bevorzugte Photoinitiatoren bzw. Photoinitiator-Systeme sind bzw. enthalten beispielsweise Campherchinon, 1-Phenyl propan-1,2 dion, Benzildiacetyl, Benzyldimethylketal, Benzyldiethylketal, Benzyldi(2-methoxyethyl)ketal, Anthrachinon, 1-Chloranthrachinon, 1-Chloranthrachinon, 1,2-Benzanthrachinon, 1-Hydroxyanthrachinon, 1-Methylanthrachinon, 2-Ethylanthrachinon, 1-Bromanthrachinon, Thioxanthon, 2-Isopropylthioxanthon, 2-Nitrothioxanthon, 2-Methylthioxanthon, 2,4-Dimethylthioxanthon, 2,4-Diethylthioxanthon, 2,4-Diisopropylthioxanthon, 2-Chlor-7-trifluormethylthioxanthon, Thioxanthon-10,10-dioxid, Thioxanthon-10-oxid, Benzoinmethylether, Benzoinethylether, Benzoinisopropylether, Benzoinisobutylether, Benzophenon, Bis(4-dimethylamino-phenyl)keton, 4,4'-Bisdiethylaminobenzophenon, Acylphosphinoxide wie 2,4,6 Trimethylbenzoyl)diphenylphosphinoxid, Bis(2,4,6-trimethylbenzoyl)-phenylphosphi-noxid, Benzoyltrimethylgermanium, Dibenzoyldiethylgermanium, und Diaryliodoniumsalze, wie Diphenyliodoniumhexafluorophosphat, Diphenyliodoniumhexafluoroantimonat, Bis(4-bromophenyl)iodoniumtriflat, Bis(4-tert-butylphenyl)iodoniumhexaflu-orophosphat, Bis(4-fluorophenyl)iodoniumtriflate, Bis(4-methylphenyl)iodoniumhexafluorophosphat, (2-Bromophenyl)(2,4,6-trimethylphenyl)iodoniumtriflat, 3-Bromophenyl)(2,4,6-trimethylphenyl)iodoniumtriflat, (2-Methylphenyl)(2,4,6-trimethylphenyl)iodoniumtriflat, (3-Methylphenyl)(2,4,6-trimethylphenyl)iodoniumtriflat, (4-Methylphenyl)(2,4,6-trimethylphenyl)iodoniumtriflat, (4-Nitrophenyl)phenyliodoniumtriflat, (4-Nitrophenyl)(2,4,6-trimethylphenyl)iodoniumtriflat, Phenyl[3-(trifluoromethyl)phenyl]iodoniumtriflat und/oder [3-(Trifluoromethyl)phenyl](2,4,6-trimethylphenyl)iodoniumtriflat. Besonders bevorzugt sind Diphenyliodoniumhexafluorophosphat oder Campherchinon.

Bevorzugte Photoinitiator-Systeme enthalten Elektronendonoren als Co-Initiator. Bevorzugte Co-Initiatoren sind tertiäre aromatische oder aliphatische Amine, bspw. N,N-Dimethylaminoethylmethacrylat oder Aminobenzoate, wie 2-Ethylhexyl-4-(dimethylamino)benzoat und Ethyl(N,N-dimethylamino)benzoat.

Besonders bevorzugte Photoinitiator-Systeme enthalten Kombinationen von Campherchinon mit mindestens einem Dimethylaminobenzoat. Weitere besonders bevorzugte Photoinitiator-Systeme sind in der WO2015124559A1 beschrieben.

In einer weiteren Ausführungsform enthält das erfindungsgemäße Dentalmaterial ein aktiviertes Initiatorsystem für die radikalische Polymerisation.

Das aktivierte Initiatorsystem für die radikalische Polymerisation ist bevorzugt bei Raumtemperatur (23°C) und noch bevorzugter bei Körpertemperatur (37°C) aktivierbar.

Aktivierte Initiatorsysteme bestehen aus mindestens einem Aktivator und mindestens einem Initiator.

Aktivator und Initiator sind zur Vermeidung von vorzeitiger Erhärtung in getrennt gelagerten Teilen des Dentalmaterials enthalten. Die getrennt gelagerten Teile des Dentalmaterials werden unmittelbar vor der Anwendung miteinander gemischt. In der Mischung führt eine Reaktion von Aktivator mit Initiator zur Initiierung der radikalischen Polymerisation und damit zur Erhärtung des Dentalmaterials.

Ein bevorzugtes aktiviertes Initiatorsystem ist ein RedOX-Initiatorsystem.

Ein bevorzugtes RedOx-Initiatorsystem ist das Amin-Peroxid-System. Das Amin-Peroxid-System enthält mindestens ein Amin als Reduktionsmittel und mindestens ein Peroxid als Oxidationsmittel. Amin-Peroxid-Systeme sind dem Fachmann bekannt und werden bspw. in DE102009005480 (Kohro, Y.), EP1444972 (Finger, W.), US7820733 (Ohara, Y.) und US7214276 (Qian, X.) beschrieben.

Bevorzugte Amine sind aliphatische tertiäre Amine, wie beispielsweise N,N-Dimethylaminoethylmethacrylat, Triethylamin, Tributylamin, Triallylamin, Triethanolamin, aromatische sekundäre und insbesondere aromatische tertiäre Amine, wie z.B. N,N-Dimethylanilin, N,N-Di(2-hydroxyethyl)-3,5-dimethylanilin, N-Methyl-N-(2-methylcarbamoyloxypropyl)-3,5-dimethylanilin, N,N-Dimethyl-p-tert-butylanilin, N,N-Dimethyl-p-toluidin, N,N-Ethyl-p-toluidin, N,N-Di(2-hydroxyethyl)-p-toluidin, N,N-Di(hydroxymethyl)-p-toluidin, N-Methyl-p-toluidin, N,N-Dimethyl-sym.-xylidin, Phenylmorpholin, Methyl-4-Dimethylaminobenzoat, Ethyl-4-Dimethylaminobenzoat, Isoamyl-4-Dimethylamino-benzoat und dergleichen. Bevorzugt sind aromatische tertiäre Amine, wie N,N-Dimethyl-p-toluidin, N,N-Di(hydroxymethyl)-p-toluidin, N,N-Dimethyl-sym.-xylidin und N,N-Dimethyl-p-tert-butylanilin.

Bevorzugte Peroxide sind bspw., Dibenzoylperoxid, 4,4'-Dichlordibenzoylperoxid, 2,4-Dichlordibenzoylperoxid, Dilauroylperoxid, tert-Butylperoxid, tert-Butylperoxybenzoat, Methylethylketonperoxid und tertiäre Hydroperoxide wie, t-Butylhydroperoxid, t-Amylhydroperoxid, p-Diisopropylbenzolhydroperoxid, Cumolhydroperoxid, Pinanhydroperoxid, p-Methanhydroperoxid, und 1,1,3,3-Tetramethylbutylhydroperoxid, 2,5-Dimethylhexan-2,5-dihydroperoxid. In bestimmten Ausführungsformen können auch anorganische Peroxide, als Beispiel seien Natriumperoxodisulfat, Kaliumperoxodisulfat, Ammoniumperoxodisulfat genannt, verwendet werden wie in EP 1 878 428 (Tokui, H.). Besonders bevorzugt sind Diacylperoxide, wie Dibenzoylperoxid und Dilauroylperoxid.

Ein weiteres bevorzugtes RedOx-Initiatorsystem enthält mindestens einen substituierten Thioharnstoff als Reduktionsmittel und mindestens ein Hydroperoxid als Oxidationsmittel. Solche RedOx-Initiatorsysteme sind dem Fachmann bekannt und werden bspw. in WO03057792 (Mitra, S.) und WO2008134024 (Liu, H.) beschrieben.

Bevorzugte substituierte Thioharnstoffe sind Benzoylthioharnstoff, 1-(2-Pyridyl)-2-thioharnstoff, 1-Acetyl-2-thioharnstoff und 1-(2-Tetrahydrofurfuryl)-2-thioharnstoff oder polymerisierbare Thioharnstoffe, wie 1-Allylthioharnstoff, 1,1-Diallylthioharnstoff, 1,3-Diallylthioharnstoff, 1-Allyl-3-(2-hydroxyethyl)-2-thioharnstoff, (Meth)acryloxyalkylthioharnstoff, 1-Allyl-3-methylthioharnstoff. Besonders bevorzugt sind Allylthioharnstoff-Derivate, 1-(2-Pyridyl)-2-thioharnstoff und 1-(2-Tetrahydrofurfuryl)-2-thioharnstoff.

Bevorzugte Hydroperoxide sind Cumolhydroperoxid, t-Butylhydroperoxid, t-Amylhydroperoxid, p-Diisopropylbenzolhydroperoxid, Pinanhydroperoxid, p-Methanhydroperoxide, und 1,1,3,3-Tetramethylbutylhydroperoxid. Besonders bevorzugt sind Cumolhydroperoxid und t-Amylhydroperoxid.

Bevorzugt weist dieses System einen sauren Promotor auf, z. B. Methacrylsäure, wie in US2003134933 (Jin, S.) beschrieben.

In einer weiteren Ausführungsform enthält das erfindungsgemäße Dentalmaterial ein Initiatorsystem, enthaltend Ascorbinsäure oder mindestens ein Derivat der Ascorbinsäure als Reduktionsmittel und mindestens ein Oxidationsmittel, wie in US5501727 (Wang, B.) und EP2371346 (Yarimizu, H.) beschrieben.

Bevorzugte Oxidationsmittel sind ausgewählt aus der Gruppen der Peroxodisulfate, wie Natrium-, Kalium-, Ammonium- und Alkylammonium-Peroxodisulfat; der Peroxide, wie Dibenzoylperoxid, Stearylperoxid, Succinsäureperoxid und der Hydroperoxide, wie Cumolhydroperoxid, tert-Butylhydroperoxid, tert-Amylhydroperoxid und 2,5-Dihydroperoxy-2,5-dimethylhexan. Besonders bevorzugt als Oxidationsmittel sind Kaliumperoxodisulfat, tert-Butylhydroperoxid und t-Amylhydroperoxid.

Bevorzugte Ascorbinsäuren und Derivate der Ascorbinsäure sind L(+) Ascorbinsäure, Dehydroascorbinsäure, Isoascorbinsäure, Tolyl-L-ascorbinsäure, 2,6-di-o-Palmitoyl-L-ascorbinsäure, D-Araboascorbinsäure, L(+)Natriumascorbat, Natriumisoascorbat und L(+)Calciumascorbat. Besonders bevorzugt sind Ammonium-, Kalium- und Natriumisoascorbat.

In einer weiteren Ausführungsform enthält das erfindungsgemäße Dentalmaterial das Initiatorsystem Sulfinat-Peroxid-System, wie in DE69829259 (Yamamoto, T.), EP2110392 (Takei, M.), WO2005035590 (Kalgutar, R.) und EP1878418 (Tokui, H.) beschrieben.

Bevorzugte Sulfinate sind aromatische Sulfinate, weiter bevorzugt die Erdalkali-, Alkali- und Ammonium-Salze aromatischer Sulfinsäuren, wie zum Beispiel der Benzolsulfinsäure, der p-Toluolsulfinsäure, o-Toluolsulfinsäure, Ethylbenzolsulfinsäure, Decylbenzolsulfinsäure, Dodecylbenzolsulfinsäure, 2,4,6-Trimethylbenzolsulfinsäure, 2,4,6-Triisopropylbenzolsulfinsäure, Chlorbenzolsulfinsäure und Naphthalensulfinsäure.

Bevorzugte Peroxide sind organische Peroxide, wie beispielsweise Diacylperoxide, Peroxyester, Dialkylperoxide, Peroxyketale, Ketonperoxide oder Hydroperoxide.

Bevorzugt sind beispielsweise Di-acetyldiperoxid, Dibenzoylperoxid, 4,4'-Dichlordibenzoylperoxid, 2,4-Dichlorodibenzoylperoxid, p,p'-Dimethoxydibenzoylperoxid, p,p'-Dimethyldiben-zoylperoxid, p,p'-Dinitrodibenzoylperoxid und m-Ditoluoylperoxid, t-Butylperoxybenzoat, Bis-t-butylperoxyisophthalat, 2,5-Dimethyl-2,5-bis(benzoylperoxy)hexan, t-Butylperoxy-2-ethylhexanoat und t-butylperoxyisopropylcarbonat, Dicumylperoxid, Dipropylperoxid, Dibutylperoxid, Di-t-butylperoxid, Dicaprylperoxid und Dilaurylperoxid, 1,1-Bis(t-butylperoxy)3,3,5-trimethylcyclohexan, 1,1-Bis(t-butylperoxy)cyclohexan und 1,1-Bis(t-hexylperoxy)cyclohexan, Methylethylketonperoxid, Cyclohexanonperoxid und Methylacetoacetatperoxid, Cumolhydroperoxid, tert-Butylhydroperoxid und tert-Amylhydroperoxid, 2,5-Dihydroperoxy-2,5-dimethylhexan.

Bevorzugte Peroxide sind anorganische Peroxide, wie beispielsweise Natriumperoxid, Kaliumperoxid, Aluminiumperoxid, Ammoniumperoxid, Ammoniumpersulfat und Kaliumpersulfat.

Besonders bevorzugt sind Dibenzoylperoxid und Natrium- oder Kaliumperoxodisulfat.

In einer weiteren Ausführungsform enthält das erfindungsgemäße Dentalmaterial als Initiator mindestens eine CH-acide Verbindung, bspw. Barbitursäure-, Thiobarbitursäurederivaten oder deren Derivate, wie beispielsweise in H. Bredereck et al., Makromol. Chem. 92, 70 (1966), US5376691 (May. U.), EP2512400 (Neffgen, St.) oder EP1194110 (Soglowek, W.) beschrieben.

In einer weiteren Ausführungsform enthält das erfindungsgemäße Dentalmaterial als Initiator mindestens ein Salz einer CH-aciden Verbindung, wie beispielsweise in EP1872767 (Lück, R.) beschrieben. Das Salz einer CH-aciden Verbindungen kann im Gegensatz zur CH-aciden Verbindung zusammen mit radikalisch polymerisierbaren Monomeren gelagert werden. Bevorzugte Initiatorsysteme, die mindestens ein Salz einer CH-aciden Verbindung enthalten, sind in der EP2198824 (Neffgen, St.) beschrieben.

In einer weiteren Ausführungsform enthält das erfindungsgemäße Dentalmaterial als Initiator zur Initiierung der radikalischen Polymerisation bevorzugt Borverbindungen, beispielsweise die in der JP2006-111584 beschrieben Borate, die nach chemischer Aktivierung durch Säuren die radikalische Polymerisation initiieren oder Borverbindungen, wie in EP1489103 (Tomikawa, T.) beschrieben. Solche, in Verbindung mit Luftsauerstoff initiierende, Borverbindungen sind beispielsweise Trialkylborane, Alkoxyalkylborane, Dialkylborane und partiell oxidierte Trialkylborane. Als Beispiele für Alkylborane seien Triethylboran, Tripropylboran, Triisopropylboran, Tributylboran, Tri-secbutylboran, Triisobutylboran, Tripentylboran, Trihexylboran, Trioctylboran, Tridecylboran, Tricyclpentylboran oder Tricyclhexylboran genannt. Bevorzugte Alkoxyalkylborane sind Butoxydibutylboran, Dialkylborane 9-Borabicyclo[3.3.1]nonan und partiell oxidierte Borverbindungen, wie partiell oxidiertes Tributylboran.

Es kann bevorzugt sein, dass das erfindungsgemäße Dentalmaterial eine Kombination aus einem oder mehreren aktivierten Initiatorsystem(en) mit einem oder mehreren Photoinitiatorsystem(en) enthält. Das Dentalmaterial ist dann dualhärtend.

### Bestandteil d) - Lösungsmittel

Das erfindungsgemäße Dentalmaterial enthält bevorzugt mindestens ein Lösungsmittel, weiter bevorzugt eine Mischung von Lösungsmitteln. Bevorzugte Lösungsmittel sind Wasser und organische Lösungsmittel. Besonders bevorzugt ist eine Mischung von Wasser und organischem Lösungsmittel.

Organische Lösungsmittel sind bevorzugt ausgewählt aus der Gruppe bestehend aus bei Raumtemperatur (23 °C) flüchtigen Lösungsmitteln mit einer nach DIN 153170 bestimmten Verdunstungszahl kleiner 80. Bevorzugte organische Lösungsmittel sind polare, weiter bevorzugt polar protische Lösungsmittel, beispielsweise Aceton, die Propanole und die Butanole. Besonders bevorzugt ist Ethanol.

### Bestandteil e) - Füllstoffe

Das erfindungsgemäße Dentalmaterial enthält bevorzugt Füllstoffe.

Bevorzugte Füllstoffe sind Glaspulver, Glaskeramikpulver, Quarzpulver, Metalloxide, Metallhydroxide, sphärische Füllstoffe wie z.B. in der DE-PS 3247800 beschrieben, amorphe Cluster-Füllstoffe wie z.B. in der WO 01/30306 beschrieben oder eine Mischung aus diesen Füllstoffen. Bevorzugte Füllstoffe sind Bariumsilikatgläser, Strontiumsilikatgläser, Borataluminosilikatgläser, Phosphataluminosilikatgläser, Fluoroaluminosilikatgläser, Calciumsilikate, Zirkonsilikate, Natriumaluminiumsilikate, Schichtsilikate, Bentonite, Zeolithe einschließlich der Molekularsiebe, die Oxide sowie die Hydroxide der Alkali- und der Erdalkalimetalle sowie Apatite.

Bevorzugte Füllstoffe sind Dentalgläser, weiter bevorzugt Dentalgläser mit einer mittleren Partikelgröße zwischen 200 nm und 50 µm.

Bevorzugte Füllstoffe sind pyrogene Kieselsäuren oder nass gefällte Kieselsäuren.

Bevorzugte Füllstoffe sind Nanofüllstoffe mit einer mittleren Partikelgröße < 100 nm. Bevorzugte Nanofüllstoffe sind nicht aggregiert und/oder nicht agglomeriert.

Bevorzugte Füllstoffe weisen an ihrer Oberfläche Acrylat- und/oder Methacrylatgruppen auf.

### Bestandteil f) - dentalübliche Additive

Das erfindungsgemäße Dentalmaterial enthält bevorzugt dentalübliche Additive. Bevorzugte Additive sind Inhibitoren, Stabilisatoren, Beschleuniger, Farbstoffe, Fluoridierungsmittel, Remineralisierungsmittel, zusätzliche Röntgenopaker und zusätzliche Filmbildner.

Der Anteil der radikalisch polymerisierbaren Verbindung nach Formel I (Bestandteil a)) an der Gesamtmasse des erfindungsgemäßen Dentalmaterials beträgt bevorzugt 1-50 Gew.-%, weiter bevorzugt 5-50 Gew.-%.

Der Anteil radikalisch copolymerisierbarer Monomere b) an der Gesamtmasse des erfindungsgemäßen Dentalmaterials beträgt bevorzugt 5-99 Gew.-%, weiter bevorzugt 5-94 Gew.-%, weiter bevorzugt 35-94 Gew.-%.

Der Anteil des Initiators für die radikalische Polymerisation c) an der Gesamtmasse des erfindungsgemäßen Dentalmaterials beträgt bevorzugt 0,01-10 Gew.-%, weiter bevorzugt 0,01-5 Gew.-%.

Der Anteil der Füllstoffe d) an der Gesamtmasse des erfindungsgemäßen Dentalmaterials beträgt bevorzugt 0-90 Gew.-%. Weiter bevorzugt sind Untergrenzen des Füllstoffanteils an der Gesamtmasse des erfindungsgemäßen Dentalmaterials von 0,1 und 1 Gew.-%. Weiter bevorzugt sind Obergrenzen des Füllstoffanteils an der Gesamtmasse des erfindungsgemäßen Dentalmaterials von 80, 65, 45 und 25 Gew.-%.

Der Lösungsmittelanteil e) an der Gesamtmasse des erfindungsgemäßen Dentalmaterials beträgt bevorzugt 0-90 Gew.-%.

Bevorzugte Untergrenzen des Wasseranteils an der Gesamtmasse des erfindungsgemäßen Dentalmaterials sind 0,0; 0,1; 1 und 5 Gew.-%. Bevorzugte Obergrenzen des Wasseranteils an der Gesamtmasse des erfindungsgemäßen Dentalmaterials sind 90, 50 und 15 Gew.-%.

Bevorzugte Untergrenzen des Anteils organischen Lösungsmittels an der Gesamtmasse des erfindungsgemäßen Dentalmaterials sind 0,0; 0,1; 1 und 5 Gew.-%. Bevorzugte Obergrenzen des Anteils organischen Lösungsmittels an der Gesamtmasse des erfindungsgemäßen Dentalmaterials sind 90, 50 und 20 Gew.-%.

Der Anteil dentalüblicher Additive f) an der Gesamtmasse des erfindungsgemäßen Dentalmaterials beträgt bevorzugt 0,01-10 Gew.-%.

Die Erfindung wird nachfolgend anhand von Beispielen erläutert.

### Herstellungsbeispiele

### I) Herstellung elektrophiler Starterverbindungen für die kationische Polymerisation von 2-Ethyl-2-oxazolin

### Starterverbindung A (11-Bromundecanbisphosphonsäuretetraethylester)

Unter Stickstoff wurden 1,4 g Natriumhydrid (60 % in Mineralöl, 35 mmol) in 15 ml trockenem Tetrahydrofuran (THF) suspendiert. Die Suspension wurde in einem Eisbad auf 0 - 5°C heruntergekühlt und 8,5 g (30 mmol) Tetraethylmethylenbisphosphonsäureester hinzugetropft. 44,4 g (148 mmol) 1,10-Dibromdekan wurden in 40 ml trockenem THF gelöst und dem Reaktionsgemisch zugesetzt. Die Lösung wurde 72 h bei RT gerührt und dann mit 30 ml wässriger Natriumhydrogencarbonat-Lösung (0,1 mol/l) versetzt. Anschließend wurde das THF abrotiert und die wässrige Phase 2x mit Toluol ausgeschüttelt. Die vereinigten organischen Phasen wurden einrotiert. Mit einer Säulen-Filtration (SiO2, 1. Eluent: Heptan/Ethylacetat 1:1, v/v; 2. Eluent: Ethanol) wurde dann das überschüssige Dibromdekan abgetrennt. Das Rohprodukt wurde mit Hilfe einer Flashchromatographie aufgereinigt (SiO2; Ethylacetat/Ethanol, 95:5, v/v). Es wurde ein gelbliches Öl erhalten (Ausbeute: 65 %).

1H-NMR (CDC13, 300 MHz): δ = 1,20-1,38(m, 10H, CH2), 1,34(t, 12H, CH3), 1,38-1,48(m, 2H, CH2), 1,48-1,62(m, 2H, CH2), 1,72-2,02(m, 4H, CH2), 2,27(tt, 1H, CH), 3,41(t, 2H, CH2Br), 4,03-4,26(m, 8H, POCH2).

### Starterverbindung B (1,13-Di-Bromtridecan-7,7-bisphosphonsäuretetraethylester)

Unter Stickstoff wurden 1,6 g Natriumhydrid (60 % in Mineralöl, 40 mmol) in 10 ml trockenem Tetrahydrofuran (THF) suspendiert. Die Suspension wurde in einem Eisbad auf 0 - 5°C heruntergekühlt und 4,7 g (16 mmol) Tetraethylmethylenbisphosphonsäureester hinzugetropft. 40 g (164 mmol) 1,6-Dibromhexan wurden in 25 ml trockenem THF gelöst und dem Reaktionsgemisch zugesetzt. Die Lösung wurde 72 h bei RT gerührt und dann mit 20 ml wässriger Natriumhydrogencarbonat-Lösung (0,1 mol/l) versetzt. Die anschließende Behandlung erfolgte analog zu Starterverbindung A. Es wurde ein gelbliches Öl erhalten (Ausbeute: 21 %).

1H-NMR (CDC13, 300 MHz): δ = 1,20-1,38(m, 4H, CH2), 1,34(t, 12H, CH3), 1,40-1,62(m, 8H, CH2), 1,72-2,02(m, 8H, CH2), 3,41(t, 4H, CH2Br), 4,03-4,26(m, 8H, POCH2).

### II) Kationische Polymerisation von 2-Ethyl-2-oxazolin

Unter Stickstoff wurden frisch destilliertes 2-Ethyl-2-oxazolin und die jeweilige Starterverbindung entsprechend Tabelle 1 in 10 oder 15 ml trockenem Acetonitril gelöst. Die Lösungen wurden in einer Synthesemikrowelle (CEM Discovery; Mikrowellenleistung: 100 W) unter Druck 10 min bei 140°C polymerisiert.

### III) Abbruch der kationischen Polymerisation mittels nucleophiler Verbindungen

### III.1) Abbruch mit Amin

Nach dem Abkühlen wurde in großem Überschuss (bezogen auf den Initiator) n-Propylamin zur Polymerlösung gegeben und dann 24 h bei 70°C unter Stickstoff und unter Rückfluss gekocht. Anschließend wurde einrotiert und der Rückstand in 10 oder 15 ml Dichlormethan aufgenommen. Die Dichlormethan-Lösung wurde zuerst mit 10 ml Natriumhydrogencarbonatlösung (c(NaHCO3) = 1 mol/l), dann mit 10 ml Wasser ausgeschüttelt. Die organische Phase wurde einrotiert und anschließend der Rückstand mit wenig Acetonitril wieder aufgenommen. Die Lösung wurde anschließend in kaltem Ether gefällt. Die Fällung wurde 2x wiederholt. Der ausgefällte weiße Feststoff wurde im Hochvakuum getrocknet.

### III.2) Abbruch mit wässriger Kaliumcarbonatlösung

Nach dem Abkühlen wurden 10 ml einer Kaliumcarbonat-Lösung (c = 10 Gewichts-%) zur Polymerlösung gegeben und dann 10 h bei 100°C unter Rückfluss gekocht. Die anschließende Behandlung erfolgte analog zu 3.1.

### IV) Hydrolyse des Poly(2-Ethyl-2-oxazolin) zu Polyethylenimin

Jeweils 4 g Polymerpulver wurden mit 100 ml halbkonzentrierte Salzsäure 24 h unter Rückfluss gekocht. Anschließend wurde die Salzsäure unter Vakuum abdestilliert und der Rückstand zweimal mit Methanol gewaschen und filtriert. Der Filterrückstand wurde im Hochvakuum getrocknet. Es wurde ein weißer Feststoff erhalten.

### V) Derivatisierung des Polyethylenimin

### V.1) Derivatisierung mit Acrylsäurechlorid

Jeweils 1 g einer Poly(ethylenimin)alkanbisphosphonsäure wurde mit 5 ml Wasser versetzt und mit NaOH-Lösung auf einen pH-Wert von 9 gestellt. Nach Abkühlen der Lösung auf 0 - 5°C wurde innerhalb von 30 Minuten zwei Drittel der in Tabelle 1 angegebenen Menge an Acrylsäurechlorid hinzugegeben. Danach wurde die Lösung auf RT erwärmt und 3 h gerührt. Anschließend wurde die Lösung auf einen pH-Wert von 11-12 gestellt, nochmals auf 0 - 5°C abgekühlt und ein Drittel der in Tabelle 1 angegebenen Menge an Acrylsäurechlorid hinzugegeben. Nach 3 h Rühren bei RT wurde die Lösung mit konzentrierter Salzsäure auf einen pH-Wert von 1-2 gestellt. Die wässrige Lösung wurde mit MEHQ stabilisiert, eingeengt und im Hochvakuum getrocknet. Der Rückstand wurde mit Ethanol aufgenommen, die Lösung filtriert und das Filtrat eingeengt. Anschließend wurde der beim Einengen anfallende Feststoff in Diethylether suspendiert, die Suspension filtriert und der Rückstand im Hochvakuum getrocknet. Es wurde ein weißer Feststoff erhalten.

### V.2) Derivatisierung mit Acrylsäurechlorid und Decansäurechlorid

Jeweils 1 g einer Poly(ethylenimin)alkanbisphosphonsäure wurden mit 5 ml Wasser versetzt und mit NaOH-Lösung auf einen pH-Wert von 9 gestellt. Nach Abkühlen der Lösung auf 0 - 5°C wurden innerhalb von 30 Minuten zwei Drittel der in Tabelle 1 angegebenen Menge an Acrylsäurechlorid hinzugegeben. Danach wurde die Lösung auf RT erwärmt und 3 h gerührt. Anschließend wurde die Lösung auf pH = 11-12 gestellt, nochmals auf 0 - 5°C abgekühlt und die in Tabelle 1 angegebene Menge Decansäurechlorid hinzugegeben. Nach Beendigung der Zugabe wurde die Lösung auf RT erwärmt und 3 h gerührt. Anschließend wurde die Lösung bei einem pH-Wert von 11-12 wieder auf 0 - 5°C abgekühlt und ein Drittel der in Tabelle 1 angegebenen Menge an Acrylsäurechlorid hinzugegeben. Nach 3 h Rühren bei RT wurde die Lösung mit konzentrierter Salzsäure auf einen pH-Wert von 1-2 gestellt. Die wässrige Lösung wurde mit MEHQ stabilisiert, eingeengt und im Hochvakuum getrocknet. Der Rückstand wurde mit Ethanol aufgenommen, die Lösung filtriert und das Filtrat eingeengt. Abschließend wurde der beim Einengen anfallende Feststoff in Diethylether suspendiert, die Suspension filtriert und der Rückstand HV getrocknet. Es wurde ein weißer Feststoff erhalten.

Es wurden folgende Verbindungen (p und r siehe Tabelle 2) erhalten:

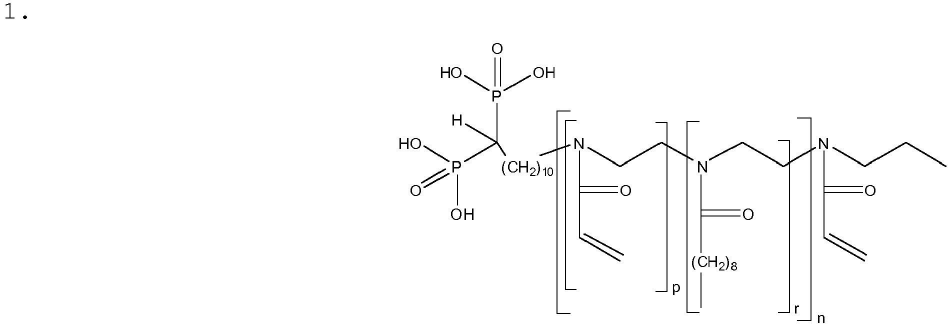

### Beispiele 1, 2, 3

1H-NMR (D2O, 300 MHz: δ = 0,85-0,95(3H, CH3), 1,18-1,38(12H, CH2), 1,45-1,95(8H, CH2), 2,05-2,35(1H, CHP), 2,6-4,2 (4H, CONCH2), 5,60-5,95 (1H, CH2CH), 5,95-6,27 (1H, CH2CH), 6,27-6,80 (1H, CH2CH).

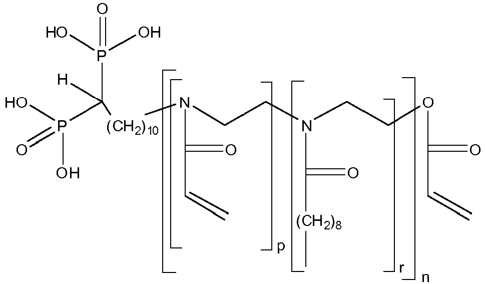

### Beispiel 5

1H-NMR (D2O, 300 MHz): δ = 1,20-1,50(12H, CH2), 1,50-1,65(2H, CH2) 1,65-2,00(4H, CH2), 2,10-2,40(1H, CHP), 2,6-4,2 (4H, CONCH2), 5,70-6,00 (1H, CH2CH), 6,00-6,35 (1H, CH2CH), 6,35-6,80 (1H, CH2CH).

### Beispiel 6

1H-NMR (DMSO, 300 MHz): δ = 0,86(3H, CH3), 1,15-1,38(24H, CH2), 1,38-1,60(6H, CH2), 1,60-1,80(2H, CH2), 2,10-2,40(2H, COCH2), 2,8-4,3 (4H, CONCH2), 5,55-5,80 (1H, CH2CH), 6,0-6,30 (1H, CH2CH), 6,45-6,90 (1H, CH2CH).

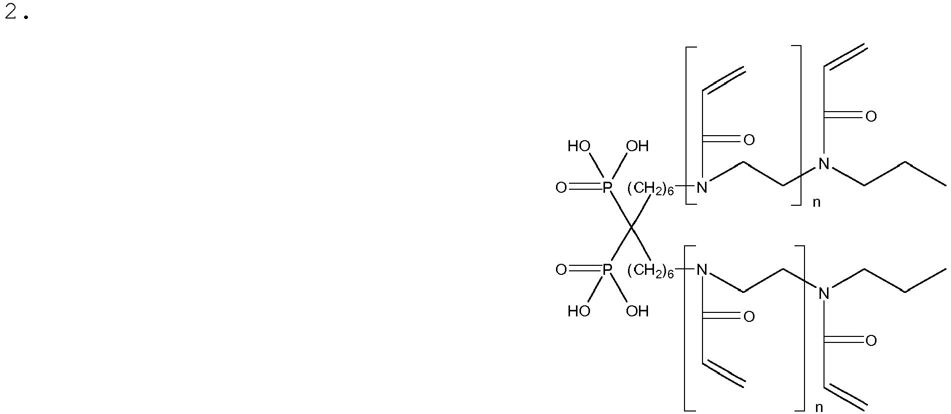

### Beispiel 4

1H-NMR (D2O, 300 MHz): δ = 0,85-0,95(6H, CH3), 1,1-1,95(24H, CH2), 2,6-4,2 (8H, CONCH2), 5,6-5,95 (2H, CH2CH), 5,98-6,27 (2H, CH2CH), 6,30-6,80 (2H, CH2CH).

**Tabelle 1**

| **Bsp.** | **Starter** | **Starter [mmol]** | **Oxazolin [mmol]** | **Abbruchverbindung** | nₜₕ | **Ausbeute [Gew. -%]** | **n_{NMR}** | **Derivatisierungsverbindung** | **Derivatisierungsverbindung [mmol]** |
|---|---|---|---|---|---|---|---|---|---|
| 1 | A | 3,9 | 18,5 | n-Propylamin | 5 | 70 | 5 | Acrylsäurechlorid | 6 |
| 2 | A | 3,9 | 39 | n-Propylamin | 10 | 70 | 7 | Acrylsäurechlorid | 12 |
| 3 | A | 3,9 | 78 | n-Propylamin | 20 | 52 | 14 | Acrylsäurechlorid | 24 |
| 4 | B | 1,2 | 24 | n-Propylamin | 10 | 37 | 8 | Acrylsäurechlorid | 24 |
| 5 | A | 3,9 | 39 | OH- | 10 | 67 | 10 | Acrylsäurechlorid | 12 |
| 6 | A | 3,9 | 39 | OH- | 10 | 67 | 10 | Acrylsäurechlorid Decansäurechlorid | 6 4 |

**Tabelle 2**

| **Bsp.** | **SG** | **x** | **A** | **n PEI** | **SK** | **p*** | **r*** | **TG** | **m** |
|---|---|---|---|---|---|---|---|---|---|
| 1 | -PO(OH)₂ | 2 | CH-(CH₂)₁₀ | 5 | -CO-CH=CH₂ | 5 | | -N(CH₂-CH₂-CH₃)(CO-CH=CH₂) | 1 |
| 2 | -PO(OH)₂ | 2 | CH-(CH₂)₁₀ | 10 | -CO-CH=CH₂ | 10 | | -N(CH₂-CH₂-CH₃)(CO-CH=CH₂) | 1 |
| 3 | -PO(OH)₂ | 2 | CH-(CH₂)₁₀ | 20 | -CO-CH=CH₂ | 20 | | -N(CH₂-CH₂-CH₃)(CO-CH=CH₂) | 1 |
| 4 | -PO(OH) ₂ | 2 | (CH₂)₆-C-(CH₂)₆ | 10 | -CO-CH=CH₂ | 10 | | -N(CH₂-CH₂-CH₃)(CO-CH=CH₂) | 2 |
| 5 | -PO(OH) ₂ | 2 | CH-(CH₂)₁₀- | 10 | -CO-CH=CH₂ | 10 | | -O-CO-CH=CH₂ | 1 |
| 6 | -PO(OH)₂ | 2 | CH-(CH₂)₁₀- | 10 | -CO-CH=CH₂ / -CO-(CH₂)₈-CH₃ | 5 | 5 | -O-CO-CH=CH₂ | 1 |

| | | | | | | | | | |
|---|---|---|---|---|---|---|---|---|---|
| * n = p + r | | | | | | | | | |

In den nachfolgenden Beispielen werden erfindungsgemäße Dentalmaterialien hergestellt und deren Hafteigenschaften anhand lichthärtender Dentaladhäsive getestet. Hierzu wurde die Scherhaftfestigkeit (SBS, Shear Bond Strength) auf Zahnschmelz ermittelt.

Zur Herstellung der lichthärtenden Dentaladhäsive wurden deren einzelnen Komponenten unter Ausschluss von die Fotopolymerisation initiierendem Licht durch Rühren bei Raumtemperatur solange vermischt bis eine homogene Lösung entstanden war.

Zur Ermittlung der Scherhaftfestigkeit (SBS) wurden Rinder-Schneidezähne ohne Pulpen in ein kalt polymerisierendes Harz eingebettet (Viscovoss GTS mit MEKP MEH-Härter; Voss Chemie). Unmittelbar vor Gebrauch wurden die eingebetteten Zähne bis zum Schmelz nass abgeschliffen (Schleifpapier P120) und anschließend mit einem feinen Schleifpapier (P500) nass nachgeschliffen. Bis zur Verwendung wurden die Zähne in demineralisiertem Wasser gelagert. Zur Messung wurden die Zähne dem demineralisierten Wasser entnommen und die Feuchtigkeit von der beschliffenen Oberfläche mit ölfreier Druckluft entfernt. Der Haftvermittler wurde mit einem Micro-Pinsel aufgetragen und 10 s einmassiert. Nach 20 s Einwirkzeit wurden die Lösungsmittel vorsichtig verblasen und die Oberfläche 10 s mit einer Dentallampe (MiniLED, ACTEON Germany, Mettmann, Deutschland) belichtet.

Anschließend wurde eine zweiteilige Teflonform mit einer Bohrung von 3,0 mm Durchmesser (ISO/TS 11405:2003) aufgesetzt, diese mit einer Metallklammer fixiert, die Kavität mit einem Dentalkomposit (Ecusit, DMG, Hamburg, Deutschland) gefüllt und 40 s belichtet (MiniLED). Nach Aushärtung wurde die Teflonform entfernt und überstehende Reste des ausgehärteten Haftvermittlers mit einem Skalpell entfernt. Die präparierten Prüfkörper wurden 23 h bei 37°C und 1h bei 23°C gelagert. Die Prüfkörper wurden dann mit einer Abschervorrichtung gemäß ISO10477:2004 und in einer Apparatur zur Bestimmung eines Kraft-Weg-Diagramms (Z010/TN2A, Zwick GmbH, & Co, Ulm, Deutschland) bei einem Vorschub von 0,5 mm/min vermessen. Das Ergebnis wird in Form eines Mittelwertes mit Standardabweichung angegeben. Die Prüfung wurde jeweils an 10 Prüfkörpern durchgeführt.

**Tabelle 3 Zusammensetzungen der lichthärtenden Dentaladhäsive sowie die ermittelten Werte der Scherhaftfestigkeit (SBS)**

| | Zusammensetzung 1 [Gew.-%] | Zusammensetzung 2 [Gew.-%] |
|---|---|---|
| Bsp. 2 | 10, 6 | 0 |
| Bsp. 5 | 0 | 10,6 |
| BisGMA | 24,8 | 24,8 |
| HEMA | 29,6 | 29,6 |
| Ethanol | 19,8 | 19,8 |
| Wasser | 13 | 13 |
| CQ | 0,8 | 0,8 |
| EHA | 1,4 | 1,4 |
| BHT | 0,01 | 0,01 |
| SBS [MPa] | 12,1 ± 3,6 | 16,5 ± 6,8 |

| | | |
|---|---|---|
| BisGMA = Bisphenol-A-diglycidyldimethacrylat; HEMA = 2-Hydroxyethylmethacrylat; CQ = Campherchinon; EHA = 2-Ethylhexyl-4-(dimethylamino)benzoate; BHT = 3,5-Di-tert-butyl-4-hydroxytoluol | | |

## Patentansprüche

1. Radikalisch polymerisierbare Verbindung der Formel I:
(SG)ₓA((PEI)TG)ₘ
mit
SG = -OPO₃R¹₂ oder -PO₃R¹₂;
R¹ = unabhängig voneinander H, C₁-C₇-Alkyl oder monovalentes Kation;
x = 2;
A: Kohlenwasserstoffgruppe mit 1 bis 30 Kohlenstoffatomen, die Silicium, Halogen, Stickstoff, Phosphor, Sauerstoff und Schwefel enthalten kann; 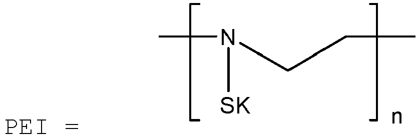
SK = H, C₁-C₂₀-Alkyl, Aryl, Alkyl-Aryl, -(CO)NR³R⁴, - (CS) NR³R⁴, -(CO)OR³, -(CO)R³ oder -(SO₂)R³;
R³, R⁴ = unabhängig voneinander H, Alkyl, Aryl und/oder Alkyl-Aryl, welche Halogen, Stickstoff, Phosphor, Sauerstoff und Schwefel enthalten können oder Alkenyl;
n = 2 - 100;
TG = H, -NR⁶R⁷, -N₃, -OR⁶, -SR⁶, Cycloalkene, -CR⁶R⁷R⁸, -OCOR⁹ oder -NR⁶COR⁹;
R⁶, R⁷, R⁸ = H, C₁ bis C₂₀-Alkyl, Aryl und/oder Alkyl-Aryl, welche Halogen, Stickstoff, Phosphor, Sauerstoff und Schwefel enthalten können; eine radikalisch polymerisierbare Gruppe, die Halogen, Stickstoff, Phosphor, Sauerstoff und Schwefel enthalten kann; wobei R⁶ und R⁷ unter Einschluss des Stickstoffatoms einen Ring mit 5 bis 7 Ringatomen bilden können, der Heteroatome enthalten kann;
R⁹ = Alkyl, Aryl, Alkyl-Aryl oder Alkenyl;
wobei PEI mehrere radikalisch polymerisierbare Gruppen enthält und TG optional mindestens eine radikalisch polymerisierbare Gruppe enthält;
m = 1, 2 oder 3.

2. Verbindung nach Anspruch 1, **dadurch gekennzeichnet, dass** SG ausgewählt ist aus -PO₃R¹₂.

3. Verbindung nach Anspruch 1 oder 2, **dadurch gekennzeichnet, dass** A eine Alkylen- oder Aryl-Alkylen- bzw. Alkyl-Arylengruppe ist, bevorzugt mit 1 bis 20 Kohlenstoffatomen, weiter bevorzugt mit 6 bis 20 Kohlenstoffatomen, besonders bevorzugt eine lineare C₂-C₁₄-Alkylengruppe.

4. Verbindung nach einem der Ansprüche 1 bis 3, **dadurch gekennzeichnet, dass** wenigstens 50%, weiter vorzugsweise wenigstens 80 %, weiter vorzugsweise wenigstens 90 %, weiter vorzugsweise alle SK ausgewählt sind aus der Gruppe bestehend aus -(SO₂)R³ und -(CO)R³, bevorzugt - (CO)R³.

5. Verbindung nach einem der Ansprüche 1 bis 4, **dadurch gekennzeichnet, dass** R³, R⁴ ausgewählt sind aus der Gruppe bestehend aus C₁ bis C₁₉-Alkyl-, bevorzugt C₅ bis C₁₀-Alkyl und C₂-C₁₉-Alkenyl, bevorzugt C₂ bis C₉-Alkenyl, weiter bevorzugt -CR⁵CH₂; mit R⁵ = H oder C₁-C₇-Alkyl, bevorzugt H oder -CH₃, besonders bevorzugt H.

6. Verbindung nach einem der Ansprüche 1 bis 5, **dadurch gekennzeichnet, dass** SK keine Säuregruppe SG aufweist.

7. Verbindung nach Anspruch 3 der Formel II: 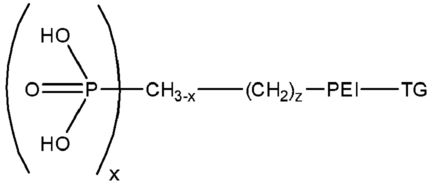 mit
x = 2;
z = 2 bis 14.

8. Verbindung nach Anspruch 3 der Formel III: 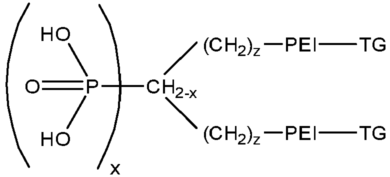 mit
x = 2;
z = 2 bis 14, bevorzugt 2 bis 9 oder weiter bevorzugt 2 bis 6.

9. Verfahren zur Herstellung einer Verbindung nach einem der Ansprüche 1 bis 8, **dadurch gekennzeichnet, dass** eine kationische Polymerisation von Oxazolinen erfolgt.

10. Verfahren nach Anspruch 9, **dadurch gekennzeichnet, dass** im Anschluss an die kationische Polymerisation eine Hydrolyse des entstandenen Polyoxazolins erfolgt, bevorzugt gefolgt von einer Derivatisierung des entstandenen Polyethylenimins zur Einführung der Seitenketten SK.

11. Verfahren nach Anspruch 9 oder 10, **dadurch gekennzeichnet, dass** über den Polymerisationsinitiator der kationischen Polymerisation die Säuregruppe SG eingebracht wird; und das über die Abbruchverbindung der kationische Polymerisation die Endgruppe(n) TG eingebracht wird/werden.

12. Verfahren nach Anspruch 9 oder 10, **dadurch gekennzeichnet, dass** über den Polymerisationsinitiator der kationischen Polymerisation die Endgruppe(n) TG eingebracht wird/werden; und das über die Abbruchverbindung der kationische Polymerisation die Säuregruppe SG eingebracht wird.

13. Verwendung einer Verbindung nach einem der Ansprüche 1 bis 8 als Bestandteil eines radikalisch polymerisierbaren Dentalmaterials.

14. Dentalmaterial, das enthält:
a) mindestens eine Verbindung nach einem der Ansprüche 1 bis 8;
b) mindestens ein mit a) radikalisch copolymerisierbares Monomer;
c) wenigstens einen Initiator für die radikalische Polymerisation;
d) optional Lösungsmittel;
e) optional Füllstoffe;
f) dentalübliche Additive.

15. Dentalmaterial nach Anspruch 14, **dadurch gekennzeichnet, dass** der Anteil der Komponenten an der Gesamtmasse wie folgt ist:
Komponente a) 1-50 Gew.-%, bevorzugt 5-50 Gew.-%;
Komponente b): 5-99 Gew.-%, bevorzugt 5-94 Gew.-%, weiter bevorzugt 35-94 Gew.-%;
Komponente c): 0,01-10 Gew.-%, bevorzugt 0,01-5 Gew.- % ;
Komponente d): wenigstens 0,1 Gew.-%, vorzugsweise wenigstens 1 Gew.-%; höchstens 80 Gew.-%, vorzugsweise höchstens 65 Gew.-%, weiter vorzugsweise höchstens 45 Gew.-%, weiter vorzugsweise höchstens 25 Gew.-%;
Komponente e): 0-90 Gew.-%.

## Claims

1. Radically polymerizable compound of the formula I:
(SG)ₓA((PEI)TG)ₘ
where
SG = -OPO₃R¹₂ or -PO₃R¹₂;
R¹ = each independently H, C₁-C₇-alkyl or monovalent cation;
x = 2;
A: hydrocarbon group having 1 to 30 carbon atoms which may comprise silicon, halogen, nitrogen, phosphorus, oxygen and sulfur; 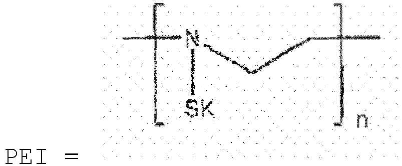
SK = H, C₁-C₂₀-alkyl, aryl, alkyl-aryl, -(CO)NR₃R₄, - (CS)NR₃R₄, -(CO)OR³, -(CO)R³ or -(SO₂)R³;
R³, R⁴ = each independently H, alkyl, aryl and/or alkyl-aryl, which may comprise halogen, nitrogen, phosphorus, oxygen and sulfur, or alkenyl;
n = 2 - 100;
TG = H, -NR⁶R⁷, -N₃, -OR⁶, -SR⁶, cycloalkenes, - CR⁶R⁷R⁸, -OCOR⁹ or -NR⁶COR⁹;
R⁶, R⁷, R⁸ = H, C₁ to C₂₀-alkyl, aryl and/or alkyl-aryl, which may comprise halogen, nitrogen, phosphorus, oxygen and sulfur; a radically polymerizable group, which may comprise halogen, nitrogen, phosphorus, oxygen and sulfur; wherein R⁶ and R⁷, with inclusion of the nitrogen atom, may form a ring having 5 to 7 ring atoms, which may comprise heteroatoms;
R⁹ = alkyl, aryl, alkyl-aryl or alkenyl;
wherein PEI comprises two or more radically polymerizable groups and TG optionally comprises at least one radically polymerizable group;
m = 1, 2 or 3.

2. Compound according to Claim 1, **characterized in that** SG is selected from -PO₃R¹₂.

3. Compound according to Claim 1 or 2, **characterized in that** A is an alkylene or aryl-alkylene or alkyl-arylene group, preferably having 1 to 20 carbon atoms, more preferably having 6 to 20 carbon atoms, particularly preferably a linear C₂-C₁₄-alkylene group.

4. Compound according to any of Claims 1 to 3, **characterized in that** at least 50%, more preferably at least 80%, more preferably at least 90%, more preferably all SKs are selected from the group consisting of -(SO₂)R³ and -(CO)R³, preferably - (CO)R³.

5. Compound according to any of Claims 1 to 4, **characterized in that** R³, R⁴ are selected from the group consisting of C₁ to C₁₉-alkyl, preferably C₅ to C₁₀-alkyl, and C₂ to C₁₉-alkenyl, preferably C₂ to C₉-alkenyl, more preferably -CR⁵CH₂; where R⁵ = H or C₁-C₇-alkyl, preferably H or -CH₃, particularly preferably H.

6. Compound according to any of Claims 1 to 5, **characterized in that** SK has no acid group SG.

7. Compound according to Claim 3 of the formula II: 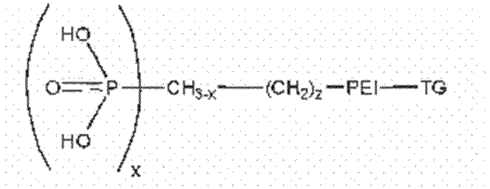 where
x = 2;
z = 2 to 14.

8. Compound according to Claim 3 of the formula III: 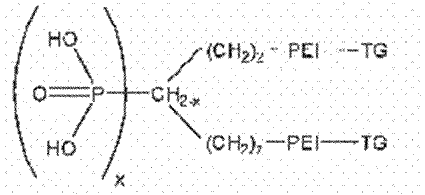 where
x = 2;
z = 2 to 14, preferably 2 to 9 or more preferably 2 to 6.

9. Process for preparing a compound according to any of Claims 1 to 8, **characterized in that** a cationic polymerization of oxazolines is carried out.

10. Process according to Claim 9, **characterized in that**, following the cationic polymerization, the resulting polyoxazoline is hydrolyzed, preferably followed by derivatization of the resulting polyethylenimine to introduce the side chains SK.

11. Process according to Claim 9 or 10, **characterized in that** the acid group SG is incorporated via the polymerization initiator of the cationic polymerization; and the end group(s) TG is/are incorporated via the termination compound of the cationic polymerization.

12. Process according to Claim 9 or 10, **characterized in that** the end group(s) TG is/are incorporated via the polymerization initiator of the cationic polymerization; and the acid group SG is incorporated via the termination compound of the cationic polymerization.

13. Use of a compound according to any of Claims 1 to 8 as a constituent of a radically polymerizable dental material.

14. Dental material comprising:
a) at least one compound according to any of Claims 1 to 8;
b) at least one monomer radically copolymerizable with a);
c) at least one initiator for the radical polymerization;
d) optionally solvents;
e) optionally fillers;
f) customary dental additives.

15. Dental material according to Claim 14, **characterized in that** the fraction of the components as a proportion of the total mass is as follows:
component a) 1-50% by weight, preferably 5-50% by weight;
component b): 5-99% by weight, preferably 5-94% by weight, more preferably 35-94% by weight;
component c): 0.01-10% by weight, preferably 0.01-5% by weight;
component d) : at least 0.1% by weight, preferably at least 1% by weight; at most 80% by weight, preferably at most 65% by weight, more preferably at most 45% by weight, more preferably at most 25% by weight;
component e): 0-90% by weight.

## Revendications

1. Composé polymérisable par voie radicalaire, de formule I :
(SG)ₓA((PEI)TG)ₘ
où
SG = -OPO₃R¹₂ ou -PO₃R¹₂ ;
R¹ = indépendamment l'un de l'autre H, un groupe alkyle en C₁-C₇ ou un cation monovalent ;
x = 2 ;
A : un groupe hydrocarboné ayant de 1 à 30 atomes de carbone, qui peut contenir des atomes de silicium, d'halogène, d'azote, de phosphore, d'oxygène et de soufre ; 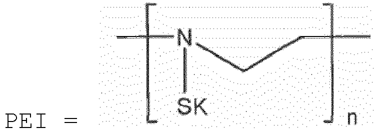
SK = H, un groupe alkyle en C₁-C₂₀, aryle, alkyl-aryle, - (CO) NR³R⁴, - (CS)NR³R⁴, -(CO)OR³, -(CO)R³ ou -(SO₂)R³ ;
R³, R⁴ = indépendamment l'un de l'autre H, un groupe alkyle, un groupe aryle et/ou un groupe alkyl-aryle, qui peuvent contenir des atomes d'halogène, d'azote, de phosphore, d'oxygène et de soufre, ou un groupe alcényle ;
n = 2 - 100 ;
TG = H, un groupe -NR⁶R⁷, -N₃, -OR⁶, -SR⁶, cycloalcène,-CR⁶R⁷R⁸, -OCOR⁹ ou -NR⁶COR⁹ ;
R⁶, R⁷, R⁸ = H, un groupe alkyle en C₁-C₂₀, un groupe aryle et/ou un groupe alkyl-aryle, qui peuvent contenir des atomes d'halogène, d'azote, de phosphore, d'oxygène et de soufre ; un groupe apte à la polymérisation radicalaire, qui peut contenir des atomes d'halogène, d'azote, de phosphore, d'oxygène et de soufre ; R⁶ et R⁷ pouvant former avec inclusion de l'atome d'azote un cycle ayant de 5 à 7 atomes formant le cycle, qui peut contenir des hétéroatomes ;
R⁹ = un groupe alkyle, aryle, alkyl-aryle ou alcényle ;
PEI contenant plusieurs groupes aptes à la polymérisation radicalaire et TG contenant en option au moins un groupe apte à la polymérisation radicalaire ;
m = 1, 2 ou 3.

2. Composé selon la revendication 1, **caractérisé en ce que** SG est choisi parmi -PO₃R¹₂.

3. Composé selon la revendication 1 ou 2, **caractérisé en ce que** A est un groupe alkylène ou aryl-alkylène ou alkyl-arylène, ayant de préférence de 1 à 20 atomes de carbone, encore mieux de 6 à 20 atomes de carbone, de façon particulièrement préférée un groupe alkylène en C₂-C₁₄ linéaire.

4. Composé selon l'une quelconque des revendications 1 à 3, **caractérisé en ce qu'**au moins 50 %, de préférence au moins 80 %, de façon particulièrement préférée au moins 90 %, de façon plus particulièrement préférée la totalité des groupes SK sont choisis dans le groupe constitué par (SO₂)R³ et -(CO)R³, de préférence -(CO)R³.

5. Composé selon l'une quelconque des revendications 1 à 4, **caractérisé en ce que** R³, R⁴ sont choisis dans le groupe constitué par les groupes alkyle en C₁-C₁₉, de préférence alkyle en C₅-C₁₀ et les groupes alcényle en C₂-C₁₉, de préférence alcényle en C₂-C₉, de façon particulièrement préférée -CR⁵CH₂ ; où R⁵ = H ou un groupe alkyle en C₁-C₇, de préférence H ou -CH₃, de façon particulièrement préférée H.

6. Composé selon l'une quelconque des revendications 1 à 5, **caractérisé en ce que** SK ne comporte pas de groupe acide.

7. Composé selon la revendication 3, de formule II : 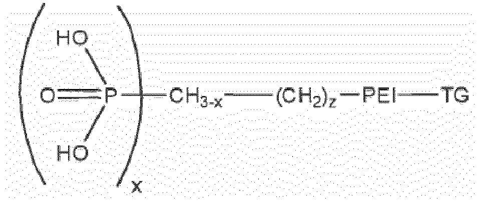 où
x = 2 ;
z = 2 à 14.

8. Composé selon la revendication 3, de formule III : 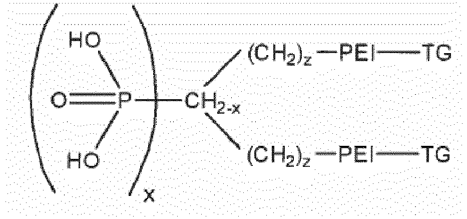 où
x = 2 ;
z = 2 à 14, de préférence 2 à 9 ou de façon particulièrement préférée 2 à 6.

9. Procédé pour la préparation d'un composé selon l'une quelconque des revendications 1 à 8, **caractérisé en ce qu'**a lieu une polymérisation cationique d'oxazolines.

10. Procédé selon la revendication 9, **caractérisé en ce qu'**à la suite de la polymérisation cationique a lieu une hydrolyse de la polyoxazoline résultante, de préférence suivie d'une transformation en dérivé de la polyéthylène-imine résultante, visant à l'introduction des chaînes latérales SK.

11. Procédé selon la revendication 9 ou 10, **caractérisé en ce que** le groupe acide SG est introduit par l'intermédiaire de l'amorceur de polymérisation de la polymérisation cationique ; et **en ce que** le(s) groupe(s) TG en bout de chaîne est/sont introduit(s) par l'intermédiaire du composé terminateur de la polymérisation cationique.

12. Procédé selon la revendication 9 ou 10, **caractérisé en ce que** le (s) groupe (s) TG en bout de chaîne est/sont introduit(s) par l'intermédiaire de l'amorceur de polymérisation de la polymérisation cationique ; et **en ce que** le groupe acide SG est introduit par l'intermédiaire du composé terminateur de la polymérisation cationique.

13. Utilisation d'un composé selon l'une quelconque des revendications 1 à 8 en tant que composant d'un matériau dentaire polymérisable par voie radicalaire.

14. Matériau dentaire, qui contient :
a) au moins un composé selon l'une quelconque des revendications 1 à 8 ;
b) au moins un monomère copolymérisable par voie radicalaire avec a) ;
c) au moins un amorceur pour la polymérisation radicalaire ;
d) en option un solvant ;
e) en option des charges ;
f) des additifs usuels pour des matériaux dentaires.

15. Matériau dentaire selon la revendication 14, **caractérisé en ce que** la proportion des composants par rapport à la masse totale est comme suit :
composant a) : 1-50 % en poids, de préférence 5-50 % en poids ;
composant b) : 5-99 % en poids, de préférence 5-94 % en poids ; de façon particulièrement préférée 35-94 % en poids ;
composant c) : 0,01-10 % en poids, de préférence 0,01-5 % en poids ;
composant d) : au moins 0,1 % en poids, de préférence au moins 1 % en poids ; au maximum 80 % en poids, de préférence au maximum 65 % en poids, de façon particulièrement préférée au maximum 45 % en poids, de façon plus particulièrement préférée au maximum 25 % en poids ;
composant e) : 0-90 % en poids.
